# FASCICULE DE BREVET EUROPEEN

(11) **EP 1 345 570 B1**
(45) Date de publication et mention de la délivrance du brevet: **05.04.2006**
(21) Numéro de dépôt: 01270304.7
(22) Date de dépôt: 11.12.2001
(51) Int. Cl.: A61Q 1/02, A61Q 1/06, A61Q 1/10

(54) **COMPOSITION COSMETIQUE COMPRENANT UNE CIRE ET UN POLYMERE**
KOSMETISCHE ZUSAMMENSETZUNG MIT EINEM WACHS UND EINEM POLYMER
COSMETIC COMPOSITION COMPRISING A WAX AND A POLYMER

(30) Priorité: 12.12.2000 FR 0016163
(43) Date de publication de la demande: 24.09.2003
(73) Titulaire: L'OREAL, 75008 Paris (FR)
(72) Inventeur: COLLIN, Nathalie, F-92330 Sceaux (FR)
(74) Mandataire: Boulard, Denis
(86) Numéro de dépôt international: PCT/FR2001/003945
(87) Numéro de publication internationale: WO 2002/047622

(56) Documents cités:
- US-A- 4 871 536
- US-A- 5 389 363
- US-A- 5 866 149
- US-A- 5 925 337

## Description

La présente invention se rapporte à une composition comprenant, dans un milieu physiologiquement acceptable, une cire et un polymère à motif amide spécifique, destinée en particulier au domaine cosmétique. L'invention se rapporte également à un procédé de maquillage ou de soin cosmétiques des matières kératiniques. La composition et le procédé de maquillage ou de soin selon l'invention sont plus particulièrement destinés aux matières kératiniques d'êtres humains telles que la peau (y compris du cuir chevelu), les ongles, les fibres kératiniques, notamment sensiblement longitudinales, telles que les cils, les sourcils et les cheveux. Plus spécialement, l'invention porte sur un mascara.

La composition selon l'invention peut se présenter sous la forme de composition de revêtement des cils (notamment de mascara), d'eye-liner, de produit pour les sourcils, de produit pour les lèvres, de fard à joues ou à paupières, de fond de teint, de produit de maquillage du corps, de produit anti-cemes, de vernis à ongles, de produit de soin de la peau, y compris du cuir chevelu, de produit pour les cheveux (mascara pour cheveux, spray).

La composition de maquillage peut également être appliquée sur les accessoires de maquillage (support) comme les, faux cils, postiches, perruques, faux ongles ou encore sur des pastilles ou patchs adhérents sur la peau ou les lèvres (du type mouches).

Les compositions de revêtement des cils, appelées mascara, comprennent généralement, de façon connue, au moins une cire sous la forme d'émulsion cire-dans-eauet au moins un polymère filmogène pour déposer un film de maquillage sur les cils et gainer ces derniers, comme le décrit par exemple le document WO-A-95/15741. Les utilisatrices attendent pour ces produits de bonnes propriétés cosmétiques telles que l'adhérence sur les cils, un allongement ou un recourbement des cils, ou bien encore une bonne tenue du mascara dans le temps, notamment une bonne résistance aux frottements par exemple des doigts ou des tissus (mouchoirs, serviettes).

Toutefois, avec ces compositions, les propriétés de maquillage comme le gainage, l'allongement ou le recourbement des cils sont obtenus lorsqu'une quantité importante de produit est déposée sur les cils à l'aide d'un applicateur, telle qu'une brosse à mascara. Lorsque la composition n'adhère pas bien sur les cils, l'utilisatrice doit appliquer plusieurs fois la brosse imprégnée de produit sur les cils, ce qui demande de consacrer un certain temps pour se maquiller et obtenir les résultats de maquillage souhaités. Or ce temps peut être perçu comme beaucoup trop long par les utilisatrices pressées. Un besoin existe donc de disposer de mascaras permettant d'obtenir rapidement et facilement le maquillage attendu.

Le but de la présente invention est de disposer d'une composition de maquillage des matières kératiniques, notamment des fibres kératiniques tels que les cils, s'appliquant facilement sur les matières kératiniques et conduisant rapidement à un maquillage présentant de bonnes propriétés cosmétiques.

Les inventeurs ont constaté de façon surprenante que l'utilisation d'un polymère à motif amide particulier dans une composition comprenant une émulsion cire-dans-eau permet d'améliorer les propriétés d'adhérence de la composition sur les matières kératiniques, notamment sur les fibres kératiniques comme les cils. La composition s'applique facilement sur les matières kératiniques et permet de déposer rapidement la composition en quantité suffisante pour obtenir un maquillage présentant les propriétés cosmétiques attendues. En particulier, on obtient rapidement un dépôt épais du maquillage sur les matières kératiniques ce qui évite aux utilisatrices d'appliquer trop longtemps la composition sur les matières kératiniques.
Ainsi, pour un mascara, on obtient un maquillage qui épaissit rapidement les fibres kératiniques, notamment les cils ; on constate ainsi une charge instantanée des cils. De plus, le mascara confère un bon allongement aux cils maquillés.

De façon plus précise, l'invention a pour objet une composition comprenant, dans un milieu aqueux physiologiquement acceptable, une émulsion de cire-dans-eau et au moins un premier polymère de masse moléculaire moyenne en poids inférieure à 100 000, comportant un squelette polymérique, ayant des motifs amide non pendant et au moins une chaîne grasse pendante et/ou au moins une chaîne grasse terminale éventuellement fonctionnalisées, ayant de 6 à 120 atomes de carbone et étant liées à ces motifs amides.

L'invention a également pour objet un procédé cosmétique de maquillage ou de soin des matières kératiniques des êtres humains comprenant l'application sur les matières kératiniques d'une composition telle que définie précédemment. De préférence, le procédé s'applique aux fibres kératiniques sensiblement longitudinales, comme les cils, les cheveux, les sourcils) et plus spécialement aux cils.

L'invention a encore pour objet l'utilisation d'une composition telle que définie précédemment pour l'obtention d'un dépôt adhérent et/ou d'un maquillage rapide sur les matières kératiniques.

L'invention a pour autre objet l'utilisation d'un mascara comprenant une composition telle que définie précédemment pour épaissir rapidement et/ou allonger les cils.

L'invention a également pour objet l'utilisation de l'association d'au moins un premier polymère de masse moléculaire moyenne en poids inférieure à 100 000 et mieux inférieure à 50 000, comportant a) un squelette polymérique ayant des motifs de répétition hydrocarbonés pourvus d'au moins un motif amide non pendant, et au moins une chaîne grasse pendante et/ou au moins une chaîne grasse terminale éventuellement fonctionnalisées, ayant de 6 à 120 atomes de carbone et étant liées à ces motifs amides, et d'au moins une cire, dans une composition physiologiquement acceptable sous forme d'émulsion cire-dans-eau,
pour l'obtention d'un dépôt adhérent sur les matières kératiniques et/ou d'un maquillage rapide des matières kératiniques et/ou pour épaissir rapidement et/ou allonger les cils.

Par milieu physiologiquement acceptable, on entend un milieu non toxique et susceptible d'être appliquée sur la peau, les phanères ou les lèvres d'êtres humains, comme un mileu cosmétique.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substituées au moins partiellement par des atomes de fluor.

Selon l'invention, ces chaînes peuvent être liées directement au squelette polymérique ou via une fonction ester ou un groupement perfluoré.

Par "polymère", on entend au sens de l'invention un composé ayant au moins 2 motifs de répétition, et de préférence au moins 3 motifs de répétition.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs motifs amides non pendants et se trouvant dans le squelette polymérique.

Avantageusement, les chaînes pendantes sont liées directement à l'un au moins des motifs amides du squelette polymérique.

Le premier polymère peut comprendre entre les motifs hydrocarbonés des motifs siliconés ou des motifs oxyalkylénés.

En outre, le premier polymère de la composition de l'invention comprend avantageusement de 40 à 98 % de chaînes grasses par rapport au nombre total des motifs amides et des chaînes grasses et mieux de 50 à 95 %. La proportion des motifs amides est fonction de la nature de la phase grasse et est en particulier similaire à la nature polaire de la phase grasse. Ainsi, plus les motifs amides sont en proportion élevée dans le premier polymère, ce qui correspond à la présence de plusieurs motifs amides, plus le premier polymère a de l'affinité avec les huiles polaires.

Avantageusement, le premier polymère de la composition selon l'invention présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000 ,et mieux de 2000 à 10 000.

Le premier polymère, et en particulier le polyamide, est non soluble dans l'eau, notamment à 25 °C. En particulier, il ne comporte pas de groupe ionique.

Comme premiers polymères préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison en particulier ester. De préférence, ces polymères comportent une chaîne grasse à chaque extrémité du squelette polyamide. Comme autre groupe de liaison on peut citer les groupes éther, amine, urée, uréthane, thioester, thiurée, thiouréthane.

Ces premiers polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone. Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I) suivante : 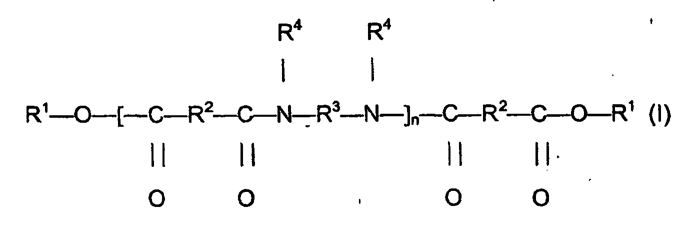 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³ avec au moins 50 % des R⁴ représentant un atome d'hydrogène.

Dans le cas particulier de la formule (I), les chaînes grasses terminales éventuellement fonctionnalisées au sens de l'invention sont des chaînes terminales liées au dernier azote du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux au moins 75 % des R² sont des groupes ayant de 30, à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂àC₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (I) se présentent sous forme de mélanges de polymères, ces mélanges pouvant en outre contenir un produit de synthèse correspondant à un composé de formule (1) où n vaut 0, c'est-à-dire un diester.

A titre d'exemple de premiers polymères selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme premier polymère utilisable dans l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Le premier polymère présent dans la composition selon l'invention a avantageusement une température de ramollissement supérieure à 65°C et pouvant aller jusqu'à 190°C. De préférence, il présente une température de ramollissement allant de 70 à 130°C et mieux de 80 à 105°C. Le premier polymère est en particulier un polymère non cireux.

De préférence, le premier polymère selon l'invention répond à la formule (I) mentionnée précédemment. Ce premier polymère présentent du fait de leur(s) chaîne(s) grasse(s), une bonne solubilité dans les huiles et donc conduisent à des compositions macroscopiquement homogènes même avec un taux élevé (au moins 25%) de polymère, contrairement à des polymères exempts de chaîne grasse.

Le premier polymère peut être présent dans la composition selon l'invention en une teneur allant de 0,01 % à 10 % en poids, par rapport au poids total de la composition, de préférence allant de 0,05 % à 5 % en poids, et mieux allant de 0,1 % à 3 % en poids.

La phase grasse de la composition selon l'invention peut comprendre une cire. Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200° C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique . On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone ou les cires fluorées.

Les cires présentes dans la composition peuvent être dispersées sous forme de particules dans un milieu aqueux. Ces particules peuvent avoir une taille moyenne allant de 50 nm à 10 µm, et de préférence de 50 nm à 3,5 µm.
En particulier, la cire peut être présente sous forme d'émulsion cires-dans-eau, les cires pouvant être sous forme de particules de taille moyenne allant de 1 µm à 10 µm, et de préférence de 1 µm à 3,5 µm.
Dans un autre mode de réalisation de la composition selon l'invention, la cire peut être présente sous forme de microdispersion de cire, la cire étant sous forme de particules dont la taille moyenne est inférieure à 1 µm, et va notamment de 50 nm à 500 nm. Des microdispersions de cires sont décrites dans les documents EP-A-557196, EP-A-1048282.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20°C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

Avantageusement, le premeir polymère et la cire peuvent être présents dans la composition selon l'invention selon un rapport pondéral cire / premier polymère allant de 5 à 60, de préférence allant de 7 à 50, et mieux de 10 à 40.

La phase aqueuse constitue la phase continue de la composition. La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄. Le solvant miscible à l'eau peut être présent en une teneur allant de 0,1 % à 20 % en poids, par rapport au poids total de la composition, et de préférence allant de 0,1 % à 10 % en poids. En particulier, la teneur en solvant organique miscible à l'eau représente de 0,1 % à 30 % du poids d'eau présente dans la composition. La phase aqueuse (eau et éventuellement le solvant organique miscible à l'eau) peut être présente, en une teneur allant de 1 % à 95 % en poids, par rapport au poids total de la composition, de préférence de 5 % à 80 % en poids, et mieux de 10 % à 60 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un deuxième polymère filmogène additionnel, différent du premier polymère décrit précédemment.

Le deuxième polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁₋C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique, Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le deuxième polymère, filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425 ®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines' d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléique;
   les muccopolysaccharides tels que l'acide hyaluronique, les chondroïtines sulfate,
   et leurs mélanges.

La taille des particules de polymères en dispersion dans la phase aqueuse peut aller de 5 nm à 600 nm, et de préférence de 20 nm à 300 nm.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les actifs cosmétiques ou dermatologiques comme par exemple des émollients, des hydratants, des vitamines, des filtres solaires, et leurs mélanges. Ces additifs peuvent être présents dans la composition en une teneur allant de 0 à 20% (notamment de 0,01 à 20 %) du poids total de la composition et mieux de 0,01 à 10% (si présents).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

L'invention est illustrée plus en détail dans les exemples suivants.

### Exemple 1 :

On a préparé un mascara ayant la composition suivante :
- Cire de carnauba 2,6 g
- Cire d'abeille 3,3 g
- Cire de paraffine 10,4 g
- Huile de jojoba hydrogénée 0,2 g
- Huile de palme hydrogénée 0,2 g
- Résine de polyamide avec groupes ester terminaux vendu sous la dénomination "UNICLEAR® 100" par la société Arizona Chemical 1 g
- Amino-2 méthyl-2 propanediol-1,3 0,8 g
- Triéthanolamine 2,4 g
- Acide stéarique 6,6 g
- Hydroxyéthylcellulose 0,8 g
- Gomme arabique 0,6 g
- Copolymère acrylate d'éthyle/méthacrylate de méthyle (80/20) en dispersion aqueuse à 50 % MA (DAITOSOL 5000 AD de SAITO) 5 g MA
- Oxyde de fer noir 7 g
- Conservateurs qs
- Eau qsp 100 g

Ce mascara s'applique facilement, adhère bien sur les cils pendant et après l'application ; ces derniers sont maquillés rapidement. Le mascara apporte une charge instantannée des cils.

### Exemple 2 :

On a préparé un mascara ayant la composition suivante :
- Cire d'abeille 7,1 g
- Huile de jojoba hydrogénée 7,1 g
- Résine de polyamide avec groupes ester terminaux vendu sous la dénomination "UNICLEAR® 100" par la société Arizona Chemical 0,5 g
- Polybutylène 1 g
- Copolymère hydroxyéthylcellulose/chlorure de di-allyl diméthyl ammonium (Celquat LOR de la société National Starch) 3,8 g
- Mono- et di-stérate de glycéryle (Tegin M de la société Goldschmidt) 2,1 g
- Monostéarate de glycéryle oxyéthyléné à 30 moles d'oxyde d'éthylène (Tagat S de la société Goldschmidt) 5,5 g
- Oxyde de fer noir 7 g
- Conservateurs qs
- Eau qsp 100 g

Ce mascara adhère bien sur les cils pendant et après l'application. Il présente une bonne charge instantannée des cils.

### Exemple 3 :

a) On a préparé une microdispersion de cire de carnauba ayant la composition suivante :
   - Cire de carnauba 27 g
   - Monostéarate de glycéryle polyoxyéthyléné (30 OE) (TAGAT S de GOLDSCHMIDT) 6,75 g
   - Ethanol 10 g
   - Eau qsp g

   On a chauffé à 90 °C la cire et le tensioactif en homogénéisant le mélange sous agitation modérée. Puis on a incorporé l'eau chauffée à 90 °C en continuant d'agiter. On a refroidit à température ambiante et ajouté l'éthanol pour obtenir une microdispersion de cire ayant un diamètre moyen de particules d'environ 170 nm.
b) On a préparé un mascara ayant la composition suivante :
   - Microdispersion de cire de l'exemple 1 5 g
   - Cire de carnauba 1,5 g
   - Cire d'abeille 3,6 g
   - Cire de paraffine 11,5 g
   - Huile de jojoba hydrogénée 0,2 g
   - Huile de palme hydrogénée 0,2 g
   - Résine de polyamide avec groupes ester terminaux vendu sous la dénomination "UNICLEAR® 100" par la société Arizona Chemical 0,5 g
   - Amino-2 méthyl-2 propanediol-1,3 0,8 g
   - Triéthanolamine 2,4 g
   - Acide stéarique 6,6 g
   - Hydroxyéthylcellulose 0,8 g
   - Gomme arabique 0,6 g
   - Copolymère acrylate d'éthyle/méthacrylate de méthyle (80/20) en dispersion aqueuse à 50 % MA (DAITOSOL 5000 AD de SAITO) 5 g MA
   - Oxyde de fer noir 7 g
   - Conservateurs qs
   - Eau qsp 100 g

Ce mascara s'applique facilement, adhère bien sur les cils pendant et après l'application ; ces derniers sont maquillés rapidement.

### Exemple 4 :

On a préparé un mascara ayant la composition suivante :
- Cire de carnauba 2,9 g
- Cire d'abeille 3,6 g
- Cire de paraffine 11,5 g
- Huile de jojoba hydrogénée 0,2 g
- Huile de palme hydrogénée 0,2 g
- Résine de polyamide avec groupes ester terminaux vendu sous la dénomination "UNICLEAR® 100" par la société Arizona Chemical 2 g
- Amino-2 méthyl-2 propanediol-1,3 0,8 g
- Triéthanolamine 2,4 g
- Acide stéarique 6,6 g
- Hydroxyéthylcellulose 0,8 g
- Gomme arabique 0,6 g
- Copolymère acrylate d'éthyle/méthacrylate de méthyle (80/20) en dispersion aqueuse à 50 % MA (DAITOSOL 5000 AD de SAITO) 2,5 g MA
- Oxyde de fer noir 7 g
- Conservateurs qs
- Eau qsp 100 g

Ce mascara s'applique facilement, adhère bien sur les cils pendant et après l'application ; ces derniers sont maquillés rapidement. On constate une charge instantannée des cils.

## Revendications

1. Composition comprenant, dans un milieu aqueux physiologiquement acceptable, une émulsion de cire-dans-eau et au moins un premier polymère de polyamide de masse moléculaire moyenne en poids inférieure à 100 000, comportant un squelette polymérique ayant des motifs répétitifs hydrocarbonés pourvus d'au moins un motif amide non pendant, et au moins une chaîne grasse pendante et/ou au moins une chaîne grasse terminale éventuellement fonctionnalisées, ayant de 8 à 120 atomes de carbone et étant liées à ces motifs amide.

2. Composition selon la revendication 1, **caractérisée par le fait que** la masse molaire moyenne du premier polymère est inférieure à 50 000.

3. Composition selon les revendications 1 ou 2, **caractérisée par le fait que** les chaînes grasses représentent de 40 à 98 % du nombre total des motifs amide et des chaînes grasses.

4. Composition selon l'une des revendications précédentes, **caractérisée par le fait que** les chaînes grasses représentent de 50 à 95 % du nombre total des motifs amide et des chaînes grasses.

5. Composition selon l'une des revendications précédentes, **caractérisée par le fait que** les chaînes grasses pendantes sont liées directement à l'un au moins des atomes d'azote des motifs amide.

6. Composition selon l'une des revendications 1 et 3 à 5, **caractérisée par le fait que** la masse molaire moyenne du premier polymère va de 1000 à 100 000.

7. Composition selon l'une des revendications 1 et 3 à 6, **caractérisée par le fait que** la masse molaire moyenne du premier polymère va de 1000 à 50 000.

8. Composition selon l'une des revendications 1 et 3 à 7, **caractérisée par le fait que** la masse molaire moyenne du premier polymère va de 1 000 à 30.000.

9. Composition selon l'une des revendications précédentes, **caractérisée par le fait que** la masse molaire moyenne en poids du premier polymère filmogène va de 2 000 à 20 000.

10. Composition selon l'une des revendications précédentes, **caractérisée par le fait que** la masse molaire moyenne en poids du premier polymère filmogène va de 2 000 à 10 000.

11. Composition selon l'une des revendications précédentes, **caractérisée par le fait que** la ou les chaînes grasses terminales sont liées au squelette par des groupes de liaison.

12. Composition selon la revendication 11, **caractérisée par le fait que** les groupes de liaison sont des groupes ester.

13. Composition selon l'une des revendications précédentes, **caractérisée par le fait que** la ou les chaînes grasses ont de 12 à 68 atomes de carbone.

14. Composition selon l'une des revendications précédentes, **caractérisée par le fait que** le premier polymère est choisi parmi les polymères de formule (I) suivante et leurs mélanges : 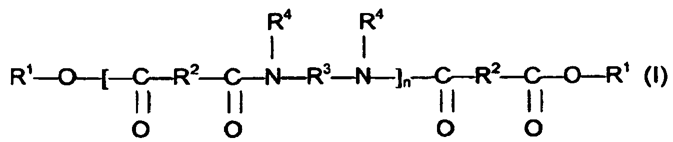 dans laquelle n désigne un nombre entier de motifs amide tel que le nombre de groupes ester représente de 10 % à 50 % du nombre total des groupes ester et amide; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse par tie d'une structure hétérocyclique définie par R⁴-N-R³, avec au moins 50 % des R⁴ représentant un atome d'hydrogène.

15. Composition selon la revendication précédente, **caractérisée par le fait que** R¹ est un groupe alkyle en C₁₂ à C₂₂.

16. Composition selon l'une des revendications 14 ou 15, **caractérisée par le fait que** R² sont des groupes ayant de 30 à 42 atomes de carbone.

17. Composition selon l'une des revendications précédentes, **caractérisée par le fait que** le premier polymère est présent en une teneur allant de 0,01 % à 10 % en poids, par rapport au poids total de la composition.

18. Composition selon l'une des revendications précédentes, **caractérisée par le fait que** le premier polymère est présent en une teneur allant de 0,05 % à 5 % en poids par rapport au poids total de la composition.

19. Composition selon l'une des revendications précédentes, **caractérisée par le fait que** le premier polymère est présent en une teneur allant 0,1 % à 3 % en poids par rapport au poids total de la composition.

20. Composition selon l'une quelconque des revendications précédentes, **caractérisée par le fait qu'**elle contient au moins une cire ayant une température de fusion supérieure à 30°C et allant jusqu'à 120 °C.

21. Composition selon l'une quelconque des revendications précédentes, **caractérisée par le fait qu'**elle contient une cire choisie dans le groupe formé par la cire d'abeilles, la cire de lanoline, les cires d'insectes de Chine, la cire de riz, la cire de Carnauba, la cire de Candellila, la cire d'Ouricury, la cire de fibres de liège, la cire de canne à sucre, la cire du Japon et la cire de sumac, la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C, les cires obtenues par hydrogénation catalytique d'huiles animales ou végétales ayant des chaînes grasses, linéaires ou ramifiées, en C8-C32, les cires de silicone, les cires fluorées, et leurs mélanges.

22. Composition selon l'une quelconque des revendications précédentes, **caractérisée par le fait qu'**elle comprend une cire ayant une dureté allant de 0,05 MPa à 15 MPa.

23. Composition selon l'une quelconque des revendications précédentes, **caractérisée par le fait que** la cire est dispersée sous forme de particules ayant une taille moyenne allant de 50 nm à 10 µm.

24. Composition selon l'une quelconque des revendications précédentes, **caractérisée par le fait que** la cire est dispersée sous forme de particules ayant une taille moyenne allant de 1 µm à 10 µm.

25. Composition selon l'une quelconque des revendications 1 à 22, **caractérisée par le fait que** la cire est dispersée sous forme de particules ayant une taille moyenne inférieure à 1 µm.

26. Composition selon l'une quelconque des revendications précédentes, **caractérisée par le fait que** la cire est présente en une teneur allant de 0,1 % à 50 % en poids, par rapport au poids total de la composition.

27. Composition selon l'une quelconque des revendications précédentes, **caractérisée par le fait qu'**elle comprend un deuxième polymère filmogène différent du premier polymère.

28. Composition selon la revendication 27, **caractérisée par le fait que** le deuxième polymère filmogène est choisi dans le groupe formé par les polymères vinyliques, les polyuréthanes, les polyesters, les polyamides, les polyurées, les polymères cellulosiques.

29. Composition selon la revendication 27 ou 28, **caractérisée par le fait que** le deuxième polymère filmogène est solubilisé dans la phase aqueuse.

30. Composition selon la revendication 27 ou 29, **caractérisée par le fait que** le deuxième polymère filmogène est sous forme de particules en dispersion aqueuse.

31. Composition selon l'une quelconque des revendications 27 à 30, **caractérisée par le fait que** deuxième polymère filmogène est présent en une teneur en matières sèches allant de 0,1 % à 60 % en poids par rapport au poids total de la composition.

32. Composition selon l'une quelconque des revendications précédentes, **caractérisée par le fait qu'**elle comprend un tensioactif émulsionnant.

33. Composition selon la revendication 32, **caractérisée par le fait que** le tensioactif émulsionnant est présent en une teneur allant de 2 à 30 % en poids par rapport au poids total de la composition.

34. Composition selon l'une quelconque des revendications précédentes, **caractérisée par le fait que** la phase aqueuse contient un solvant organique miscible avec l'eau.

35. Composition selon la revendication 34, **caractérisée par le fait que** le solvant organique miscible à l'eau est choisi dans le groupe formé par les monoalcools inférieurs ayant de 1 à 5 atomes de carbone, les glycols ayant de 2 à 8 atomes de carbone, les cétones en C₃-C₄, les aldéhydes en C₂-C₄.

36. Composition selon la revendication 34 ou 35, **caractérisée par le fait que** le solvant organique miscible à l'eau est choisi dans le groupe formé par l'éthanol, l'isopropanol, le propylène glycol, l'éthylène glycol, le 1,3-butylène glycol, le dipropylène glycol.

37. Composition selon l'une quelconque des revendications 34 à 36, **caractérisée par le fait que** le solvant organique miscible à l'eau est présent en une teneur allant de 0,1 % à 20 % en poids, par rapport au poids total de la composition.

38. Composition selon l'une quelconque des revendications précédentes, **caractérisée par le fait que** la phase aqueuse constituée d'eau et éventuellement de solvant organique miscible à l'eau est présente, en une teneur allant de 1 % à 95 % en poids, par rapport au poids total de la composition.

39. Composition selon l'une des revendications précédentes, **caractérisée par le fait qu'**elle contient, en outre, au moins une matière colorante.

40. Composition selon la revendication 39, **caractérisée par le fait que** la matière colorante est choisie parmi les pigments, les nacres, les colorants hydrosolubles, et leurs mélanges.

41. Composition selon la revendication 39 ou 40, **caractérisée par le fait que** la matière colorante est présente à raison de 0,01 à 50 % du poids total de la composition.

42. Composition selon l'une quelconque des revendications précédentes, **caractérisée par le fait qu'**elle comprend un agent épaississant.

43. Composition selon l'une des revendications précédentes, **caractérisée par le fait qu'**elle constitue une composition de soin ou de maquillage des matières kératiniques.

44. Composition selon l'une des revendications précédentes, **caractérisée par le fait qu'**elle contient au moins un additif choisi parmi les antioxydants, les charges, les conservateurs, les parfums, les neutralisants, les actifs cosmétiques ou dermatologiques, les huiles, et leurs mélanges.

45. Composition selon l'une des revendications précédentes, **caractérisée par le fait qu'**elle se présente sous forme de mascara, d'eye-liner, de produit pour les sourcils, de produit pour les lèvres, de fard à joues ou à paupières, de fond de teint, de produit de maquillage du corps, de produit anti-cemes, de vernis à ongles, de produit de soin de la peau, de produit pour les cheveux.

46. Mascara comprenant une composition selon l'une quelconque des revendications 1 à 45.

47. Procédé cosmétique de maquillage ou de soin des matières kératiniques des êtres humains, comprenant l'application sur les matières kératiniques d'une composition cosmétique conforme à l'une des revendications 1 à 45.

48. Utilisation d'une composition selon l'une quelconque des revendications 1 à 45 pour l'obtention d'un dépôt adhérent sur les matières kératiniques et/ou pour obtenir un maquillage rapide des matières kératiniques.

49. Utilisation d'un mascara selon la revendication 46 pour épaissir rapidement et/ou allonger les cils.

50. Utilisation de l'association d'au moins un premier polymère de masse moléculaire moyenne en poids inférieure à 100 000; comportant un squelette polymérique ayant des motifs de répétition hydrocarbonés pourvus d'au moins un motif amide non pendant, et au moins une chaîne grasse pendante et/ou au moins une chaîne grasse terminale éventuellement fonctionnalisées, ayant de 6 à 120 atomes de carbone et étant liées à ces motifs amides, et d'au moins une cire, dans une composition physiologiquement acceptable sous forme d'émulsion cire-dans-eau, pour l'obtention d'un dépôt adhérent sur les matières kératiniques et/ou d'un maquillage rapide des matières kératiniques et/ou pour épaissir rapidement et/ou allonger les cils.

51. Utilisation selon la revendication 50, **caractérisée par le fait que** le premier polymère est un polyamide comportant des groupements terminaux à groupe ester comportant une chaîne hydrocarbonée ayant de 10 à 42 atomes de carbone.

52. Utilisation selon l'une des revendications 50 ou 51, **caractérisée en ce que** le premier polymère présente une masse moléculaire moyenne en poids allant de 1000 à 30 000.

## Claims

1. Composition comprising, in a physiologically acceptable aqueous medium, a wax-in-water emulsion and at least one first polyamide polymer with a weight-average molecular mass of less than 100 000 comprising a polymer backbone having hydrocarbonaceous repeat units which are provided with at least one nonpendent amide unit and at least one pendent fatty chain and/or at least one end fatty chain, which fatty chains are optionally functionalized, have from 8 to 120 carbon atoms and are bonded to these amide units.

2. Composition according to Claim 1, **characterized in that** the average molar mass of the first polymer is less than 50 000.

3. Composition according to Claim 1 or 2, **characterized in that** the fatty chains represent from 40 to 98% of the total number of amide units and of the fatty chains.

4. Composition according to one of the preceding claims, **characterized in that** the fatty chains represent from 50 to 95% of the total number of the amide units and of the fatty chains.

5. Composition according to one of the preceding claims, **characterized in that** the pendent fatty chains are bonded directly to at least one of the nitrogen atoms of the amide units.

6. Composition according to one of Claims 1 and 3 to 5, **characterized in that** the average molar mass of the first polymer ranges from 1000 to 100 000.

7. Composition according to one of Claims 1 and 3 to 6, **characterized in that** the average molar mass of the first polymer ranges from 1000 to 50 000.

8. Composition according to one of Claims 1 and 3 to 7, **characterized in that** the average molar mass of the first polymer ranges from 1000 to 30 000.

9. Composition according to one of the preceding claims, **characterized in that** the weight-average molar mass of the first film-forming polymer ranges from 2000 to 20 000.

10. Composition according to one of the preceding claims, **characterized in that** the weight-average molar mass of the first film-forming polymer ranges from 2000 to to 10 000.

11. Composition according to one of the preceding claims, **characterized in that** the end fatty chain or chains are bonded to the backbone via bonding groups.

12. Composition according to Claim 11, **characterized in that** the bonding groups are ester groups.

13. Composition according to one of the preceding claims, **characterized in that** the fatty chain or chains have from 12 to 68 carbon atoms.

14. Composition according to one of the preceding claims, **characterized in that** the first polymer is chosen from polymers of following formula (I) and their blends: 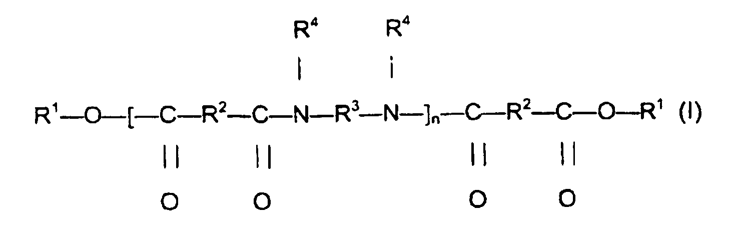 in which n denotes an integral number of amide units such that the number of ester groups represents from 10% to 50% of the total number of the ester and amide groups; R¹ is, in each case, independently an alkyl or alkenyl group having at least 4 carbon atoms; R² independently represents, in each case, a C₄ to C₄₂ hydrocarbonaceous group, provided that 50% of the R² groups represent a C₃₀ to C₄₂ hydrocarbonaceous group; R³ independently represents, in each case, an organic group provided with at least 2 carbon atoms, with hydrogen atoms and optionally with one or more oxygen or nitrogen atoms; and R⁴ independently represents, in each case, a hydrogen atom, a C₁ to C₁₀ alkyl group or a direct bond to R³ or another R⁴, so that the nitrogen atom to which both R³ and R⁴ are bonded forms part of a heterocyclic structure defined by R⁴-N-R³, with at least 50% of the R⁴ groups representing a hydrogen atom.

15. Composition according to the preceding claim, **characterized in that** R¹ is a C₁₂ to C₂₂ alkyl group.

16. Composition according to either of Claims 14 and 15, **characterized in that** R² are groups having from 30 to 42 carbon atoms.

17. Composition according to one of the preceding claims, **characterized in that** the first polymer is present in a content ranging from 0.01% to 10% by weight with respect to the total weight of the composition.

18. Composition according to one of the preceding claims, **characterized in that** the first polymer is present in a content ranging from 0.05% to 5% by weight with respect to the total weight of the composition.

19. Composition according to one of the preceding claims, **characterized in that** the first polymer is present in a content ranging from 0.1% to 3% by weight with respect to the total weight of the composition.

20. Composition according to any one of the preceding claims, **characterized in that** it comprises at least one wax having a melting temperature of greater than 30°C and ranging up to 120°C.

21. Composition according to any one of the preceding claims, **characterized in that** it comprises a wax chosen from the group formed by beeswax, lanolin wax, Chinese insect wax, rice wax, carnauba wax, candelilla wax, ouricury wax, cork fibre wax, sugar cane wax, Japan wax and sumac wax, montan wax, microcrystalline waxes, paraffin waxes, ozokerites, ceresin wax, lignite wax, polyethylene waxes, the waxes obtained by the Fischer-Tropsch synthesis, fatty acid esters and glycerides which are solid at 40°C, the waxes obtained by catalytic hydrogenation of animal or vegetable oils having linear or branched C₈-C₃₂ fatty chains, silicone waxes, fluorinated waxes, and their mixtures.

22. Composition according to any one of the preceding claims, **characterized in that** it comprises a wax having a hardness ranging from 0.05 MPa to 15 MPa.

23. Composition according to any one of the preceding claims, **characterized in that** the wax is dispersed in the form of particles having a mean size ranging from 50 nm to 10 µm.

24. Composition according to any one of the preceding claims, **characterized in that** the wax is dispersed in the form of particles having a mean size ranging from 1 µm to 10 µm.

25. Composition according to any one of Claims 1 to 22, **characterized in that** the wax is dispersed in the form of particles having a mean size of less than 1 µm.

26. Composition according to any one of the preceding claims, **characterized in that** the wax is present in a content ranging from 0.1% to 50% by weight with respect to the total weight of the composition.

27. Composition according to any one of the preceding claims, **characterized in that** it comprises a second film-forming polymer other than the first polymer.

28. Composition according to Claim 27, **characterized in that** the second film-forming polymer is chosen from the group formed by vinyl polymers, polyurethanes, polyesters, polyamides, polyureas and cellulose polymers.

29. Composition according to Claim 27 or 28, **characterized in that** the second film-forming polymer is dissolved in the aqueous phase.

30. Composition according to Claim 27 or 29, **characterized in that** the second film-forming polymer is in the form of particles in aqueous dispersion.

31. Composition according to any one of Claims 27 to 30, **characterized in that** the second film-forming polymer is present in a content on a dry basis ranging from 0.1% to 60% by weight with respect to the total weight of the composition.

32. Composition according to any one of the preceding claims, **characterized in that** it comprises an emulsifying surfactant.

33. Composition according to Claim 32, **characterized in that** the emulsifying surfactant is present in a content ranging from 2 to 30% by weight with respect to the total weight of the composition.

34. Composition according to any one of the preceding claims, **characterized in that** the aqueous phase comprises an organic solvent which is miscible with water.

35. Composition according to Claim 34, **characterized in that** the water-miscible organic solvent is chosen from the group formed by lower monoalcohols having from 1 to 5 carbon atoms, glycols having from 2 to 8 carbon atoms, C₃-C₄ ketones and C₂-C₄ aldehydes.

36. Composition according to Claim 34 or 35, **characterized in that** the water-miscible organic solvent is chosen from the group formed by ethanol, isopropanol, propylene glycol, ethylene glycol, 1,3-butylene glycol and dipropylene glycol.

37. Composition according to any one of Claims 34 to 36, **characterized in that** the water-miscible organic solvent is present in a content ranging from 0.1% to 20% by weight with respect to the total weight of the composition.

38. Composition according to any one of the preceding claims, **characterized in that** the aqueous phase composed of water and optionally of water-miscible organic solvent is present in a content ranging from 1% to 95% by weight with respect to the total weight of the composition.

39. Composition according to one of the preceding claims, **characterized in that** it additionally comprises at least one colouring material.

40. Composition according to Claim 39, **characterized in that** the colouring material is chosen from pigments, pearlescent agents, water-soluble dyes, and their mixtures.

41. Composition according to claim 39 or 40, **characterized in that** the colouring material is present in a proportion of 0.01 to 50% of the total weight of the composition.

42. Composition according to any one of the preceding claims, **characterized in that** it comprises a thickening agent.

43. Composition according to one of the preceding claims, **characterized in that** it constitutes a composition for caring for or making up keratinous substances.

44. Composition according to one of the preceding claims, **characterized in that** it comprises at least one additive chosen from antioxidants, fillers, preservatives, fragrances, neutralizing agents, cosmetic or dermatological active principles, oils, and their mixtures.

45. Composition according to one of the preceding claims, **characterized in that** it is provided in the form of a mascara, an eyeliner, a product for the eyebrows, a product for the lips, a face powder or eyeshadow, a foundation, a product for making up the body, a concealer, a nail varnish, a product for caring for the skin or a product for the hair.

46. Mascara comprising a composition according to any one of Claims 1 to 45.

47. Cosmetic process for making up or caring for human keratinous substances, comprising the application, to the keratinous substances, of a cosmetic composition in accordance with one of Claims 1 to 45.

48. Use of a composition according to any one of Claims 1 to 45 for producing an adherent layer on keratinous substances and/or for producing fast making up of keratinous substances.

49. Use of a mascara according to Claim 46 for rapidly thickening and/or for lengthening the eyelashes.

50. Use of the combination of at least one first polymer with a weight-average molecular mass of less than 100 000, comprising a polymer backbone having hydrocarbonaceous repeat units which are provided with at least one nonpendent amide unit and at least one pendent fatty chain and/or at least one end fatty chain, which fatty chains are optionally functionalized, have from 6 to 120 carbon atoms and are bonded to these amide units, and of at least one wax, in a physiologically acceptable composition in the form of a wax-in-water emulsion, for producing an adherent layer on keratinous substances and/or fast making up of keratinous substances and/or for rapidly thickening and/or for lengthening the eyelashes.

51. Use according to Claim 50, **characterized in that** the first polymer is a polyamide comprising end groups comprising an ester group comprising a hydrocarbonaceous chain having from 10 to 42 carbon atoms.

52. Use according to either of Claims 50 and 51, **characterized in that** the first polymer exhibits a weight-average molecular mass ranging from 1000 to 30 000.

## Patentansprüche

1. Zusammensetzung, die in einem physiologisch akzeptablen wässerigen Medium eine Wachs-in-Wasser-Emulsion und mindestens ein erstes Polyamidpolymer mit einer gewichtsmittleren Molmasse unter 100.000 enthält, das ein Polymergerüst auf weist, das wiederkehrende Kohlenwasserstoffeinheiten besitzt, die mindestens eine nicht als Seitenkette vorliegende Amideinheit und mindestens eine als Seitenkette vorliegende Fettkette und/oder mindestens eine endständige Fettkette aufweisen, die gegebenenfalls funktionalisiert sein können und 8 bis 120 Kohlenstoffatome aufweisen und an die Amideinheiten gebunden sind.

2. Zusammensetzung nach Anspruch 1, **dadurch gekennzeichnet, dass** die mittlere Molmasse des ersten Polymers unter 50.000 liegt.

3. Zusammensetzung nach den Ansprüchen 1 oder 2, **dadurch gekennzeichnet, dass** die Fettketten 40 bis 98 % der Gesamtzahl der Amideinheiten und Fettketten ausmachen.

4. Zusammensetzung nach einem der vorhergehenden Ansprüche, **dadurch gekennzeichnet, dass** die Fettketten 50 bis 95 % der Gesamtzahl der Amideinheiten und Fettketten ausmachen.

5. Zusammensetzung nach einem der vorhergehenden Ansprüche, **dadurch gekennzeichnet, dass** die seitlichen Fettketten direkt an mindestens ein Stickstoffatom der Amideinheiten gebunden sind.

6. Zusammensetzung nach einem der Ansprüche 1 und 3 bis 5, **dadurch gekennzeichnet, dass** die mittlere Molmasse des ersten Polymers im Bereich von 1.000 bis 100.000 liegt.

7. Zusammensetzung nach einem der Ansprüche 1 und 3 bis 6, **dadurch gekennzeichnet, dass** die mittlere Molmasse des ersten Polymers im Bereich von 1.000 bis 50.000 liegt.

8. Zusammensetzung nach einem der Ansprüche 1 und 3 bis 7, **dadurch gekennzeichnet, dass** die mittlere Molmasse des ersten Polymers im Bereich von 1.000 bis 30.000 liegt.

9. Zusammensetzung nach einem der vorhergehenden Ansprüche, **dadurch gekennzeichnet, dass** die gewichtsmittlere Molmasse des ersten filmbildenden Polymers im Bereich von 2.000 bis 20.000 liegt.

10. Zusammensetzung nach einem der vorhergehenden Ansprüche, **dadurch gekennzeichnet, dass** die gewichtsmittlere Molmasse des ersten filmbildenden Polymers im Bereich von 2.000 bis 10.000 liegt.

11. Zusammensetzung nach einem der vorhergehenden Ansprüche, **dadurch gekennzeichnet, dass** die endständige(n) Fettkette(n) über Verbindungsgruppen an das Grundgerüst gebunden sind.

12. Zusammensetzung nach Anspruch 11, **dadurch gekennzeichnet, dass** die Verbindungsgruppen Estergruppen sind.

13. Zusammensetzung nach einem der vorhergehenden Ansprüche, **dadurch gekennzeichnet, dass** die Fettkette(n) 12 bis 68 Kohlenstoffatome aufweisen.

14. Zusammensetzung nach einem der vorhergehenden Ansprüche, **dadurch gekennzeichnet, dass** das erste Polymer unter den Polymeren der folgenden Formel (I) und deren Gemischen ausgewählt ist: 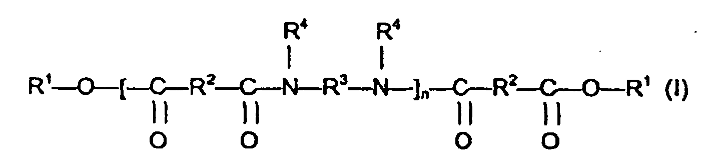 worin n eine ganze Zahl von Amideinheiten bedeutet, die so gewählt ist, dass die Anzahl der Estergruppen 10 bis 50 % der Gesamtzahl der Estergruppen und Amidgruppen ausmacht; R¹ jeweils unabhängig eine Alkyl- oder Alkenylgruppe mit mindestens 4 Kohlenstoffatomen bedeutet; R² jeweils unabhängig eine Kohlenwasserstoffgruppe mit 2 bis 42 Kohlenstoffatomen bedeutet, mit der Maßgabe, dass 50 % der Gruppen R² eine Kohlenwasserstoffgruppe mit 30 bis 42 Kohlenstoffatomen bedeuten; R³ jeweils unabhängig eine organische Gruppe ist, die mindestens 2 Kohlenstoffatome, Wasserstoffatome und gegebenenfalls ein oder mehrere Sauerstoff- oder Stickstoffatome aufweist; und R⁴ jeweils unabhängig ein Wasserstoffatom, eine C₁₋₁₀-Alkylgruppe oder eine direkte Bindung zu R³ oder einer anderen Gruppe R⁴ bedeutet, so dass das Stickstoffatom, an das die Gruppen R³ und R⁴ gleichzeitig gebunden sind, Teil einer heterocyclischen Struktur R⁴-N-R³ ist, wobei mindestens 50 % der Gruppen R⁴ ein Wasserstoffatom bedeuten.

15. Zusammensetzung nach dem vorhergehenden Anspruch, **dadurch gekennzeichnet, dass** R¹ eine C₁₂₋₂₂-Alkylgruppe ist.

16. Zusammensetzung nach einem der Ansprüche 14 oder 15, **dadurch gekennzeichnet, dass** die Gruppen R² Gruppen mit 30 bis 42 Kohlenstoffatomen sind.

17. Zusammensetzung nach einem der vorhergehenden Ansprüche, **dadurch gekennzeichnet, dass** das erste Polymer in einer Menge von 0,01 bis 10 Gew.-%, bezogen auf das Gesamtgewicht der Zusammensetzung, enthalten ist.

18. Zusammensetzung nach einem der vorhergehenden Ansprüche, **dadurch gekennzeichnet, dass** das erste Polymer in einer Menge von 0,05 bis 5 Gew.-%, bezogen auf das Gesamtgewicht der Zusammensetzung, enthalten ist.

19. Zusammensetzung nach einem der vorhergehenden Ansprüche, **dadurch gekennzeichnet, dass** das erste Polymer in einer Menge von 0,1 bis 3 Gew.-%, bezogen auf das Gesamtgewicht der Zusammensetzung, enthalten ist.

20. Zusammensetzung nach einem der vorhergehenden Ansprüche, **dadurch gekennzeichnet, dass** sie mindestens ein Wachs mit einer Schmelztemperatur über 30 °C und bis zu 120 °C enthält.

21. Zusammensetzung nach einem der vorhergehenden Ansprüche, **dadurch gekennzeichnet, dass** sie ein Wachs enthält, das ausgewählt ist unter Bienenwachs, Lanolinwachs, Chinawachsen, Reiswachs, Carnaubawachs, Candelillawachs, Ouricurywachs, Korkfaserwachs, Zuckerrohrwachs, Japanwachs, Sumachwachs, Montanwachs, mikrokristallinen Wachsen, Paraffinwachsen, Ozokeriten, Ceresin, Lignitwachs, Polyethylenwachsen, Wachsen, die durch Fischer-Tropsch-Synthese hergestellt wurden, Estern von Fettsäuren und Glyceriden, die bei 40 °C fest sind, Wachsen, die durch katalytische Hydrierung von tierischen oder pflanzlichen Ölen hergestellt sind, die geradkettige oder verzweigte Fettketten mit 8 bis 32 Kohlenstoffatomen aufweisen, Siliconwachsen, fluorierten Wachsen und deren Gemischen.

22. Zusammensetzung nach einem der vorhergehenden Ansprüche, **dadurch gekennzeichnet, dass** sie ein Wachs mit einer Härte von 0,05 bis 15 MPa enthält.

23. Zusammensetzung nach einem der vorhergehenden Ansprüche, **dadurch gekennzeichnet, dass** das Wachs in Form von Partikeln mit einer mittleren Größe von 50 nm bis 10 µm dispergiert ist.

24. Zusammensetzung nach einem der vorhergehenden Ansprüche, **dadurch gekennzeichnet, dass** das Wachs in Form von Partikeln mit einer mittleren Größe von 1 bis 10 µm dispergiert ist.

25. Zusammensetzung nach einem der Ansprüche 1 bis 22, **dadurch gekennzeichnet, dass** das Wachs in Form von Partikeln mit einer mittleren Größe unter 1 µm dispergiert ist.

26. Zusammensetzung nach einem der vorhergehenden Ansprüche, **dadurch gekennzeichnet, dass** das Wachs in einer Menge von 0,1 bis 50 Gew.-%, bezogen auf das Gesamtgewicht der Zusammensetzung, enthalten ist.

27. Zusammensetzung nach einem der vorhergehenden Ansprüche, **dadurch gekennzeichnet, dass** sie ein zweites filmbildendes Polymer enthält, das von dem ersten Polymer verschieden ist.

28. Zusammensetzung nach Anspruch 27, **dadurch gekennzeichnet, dass** das zweite filmbildende Polymer unter den Vinylpolymeren, Polyurethanen, Polyestern, Polyamiden, Polyharnstoffen und Cellulosepolymeren ausgewählt ist.

29. Zusammensetzung nach Anspruch 27 oder 28, **dadurch gekennzeichnet, dass** das zweite filmbildende Polymer in der wässerigen Phase gelöst ist.

30. Zusammensetzung nach Anspruch 27 oder 29, **dadurch gekennzeichnet, dass** das zweite filmbildende Polymer in Form von Partikeln in wässeriger Dispersion vorliegt.

31. Zusammensetzung nach einem der Ansprüche 27 bis 30, **dadurch gekennzeichnet, dass** das zweite filmbildende Polymer in einem Trockensubstanzgehalt von 0,1 bis 60 Gew.-%, bezogen auf das Gesamtgewicht der Zusammensetzung, enthalten ist.

32. Zusammensetzung nach einem der vorhergehenden Ansprüche, **dadurch gekennzeichnet, dass** sie ferner einen emulgierenden grenzflächenaktiven Stoff enthält.

33. Zusammensetzung nach Anspruch 32, **dadurch gekennzeichnet, dass** der emulgierende grenzflächenaktive Stoff in einer Menge von 2 bis 30 Gew.-%, bezogen auf das Gesamtgewicht der Zusammensetzung, enthalten ist.

34. Zusammensetzung nach einem der vorhergehenden Ansprüche, **dadurch gekennzeichnet, dass** die wässerige Fettphase ein mit Wasser mischbares organisches Lösungsmittel enthält.

35. Zusammensetzung nach Anspruch 34, **dadurch gekennzeichnet, dass** das mit Wasser mischbare organische Lösungsmittel unter den niederen Monoalkoholen mit 1 bis 5 Kohlenstoffatomen, Glykolen mit 2 bis 8 Kohlenstoffatomen, Ketonen mit 3 bis 4 Kohlenstoffatomen und Aldehyden mit 2 bis 4 Kohlenstoffatomen ausgewählt ist.

36. Zusammensetzung nach Anspruch 34 oder 35, **dadurch gekennzeichnet, dass** das mit Wasser mischbare organische Lösungsmittel unter Ethanol, Isopropanol, Propylenglykol, Ethylenglykol, 1,3-Butylenglykol und Dipropylenglykol ausgewählt ist.

37. Zusammensetzung nach einem der Ansprüche 34 bis 36, **dadurch gekennzeichnet, dass** das mit Wasser mischbare organische Lösungsmittel in einer Menge von 0,1 bis 20 Gew.-%, bezogen auf das Gesamtgewicht der Zusammensetzung, enthalten ist.

38. Zusammensetzung nach einem der vorhergehenden Ansprüche, **dadurch gekennzeichnet, dass** die wässerige Phase, die aus Wasser und gegebenenfalls einem mit Wasser mischbaren organischen Lösungsmittel besteht, in einer Menge von 1 bis 95 % Gew.-%, bezogen auf das Gesamtgewicht der Zusammensetzung, enthalten ist.

39. Zusammensetzung nach einem der vorhergehenden Ansprüche, **dadurch gekennzeichnet, dass** sie ferner mindestens ein Farbmittel enthält.

40. Zusammensetzung nach Anspruch 39, **dadurch gekennzeichnet, dass** das Farbmittel unter den Pigmenten, Perlglanzpigmenten, wasserlöslichen Farbstoffen und deren Gemischen ausgewählt ist.

41. Zusammensetzung nach Anspruch 39 oder 40, **dadurch gekennzeichnet, dass** das Farbmittel in einer Menge von 0,01 bis 50 % des Gesamtgewichts der Zusammensetzung vorliegt.

42. Zusammensetzung nach einem der vorhergehenden Ansprüche, **dadurch gekennzeichnet, dass** sie ein Verdickungsmittel enthält.

43. Zusammensetzung nach einem der vorhergehenden Ansprüche, **dadurch gekennzeichnet, dass** es sich um eine Zusammensetzung zur Pflege oder zum Schminken von Keratinsubstanzen handelt.

44. Zusammensetzung nach einem der vorhergehenden Ansprüche, **dadurch gekennzeichnet, dass** sie mindestens einen Zusatzstoff enthält, der unter den Antioxidantien, Füllstoffen, Konservierungsmitteln, Parfums, Neutralisationsmitteln, kosmetischen oder dermatologischen Wirkstoffen, Ölen und deren Gemischen ausgewählt ist.

45. Zusammensetzung nach einem der vorhergehenden Ansprüche, **dadurch gekennzeichnet, dass** sie als Mascara, Eyeliner, Produkt für die Augenbrauen, Produkt für die Lippen, Wangenrouge, Lidschatten, Make-up, Produkt zum Schminken des Körpers, Produkt gegen Augenringe, Nagellack, Produkt zur Pflege der Haut oder Produkt für die Haare vorliegt.

46. Mascara mit einer Zusammensetzung nach einem der Ansprüche 1 bis 45.

47. Kosmetisches Verfahren zum Schminken oder zur Pflege von menschlichen Keratinsubstanzen, das das Aufbringen einer kosmetischen Zusammensetzung nach einem der Ansprüche 1 bis 45 auf die Keratinsubstanzen umfasst.

48. Verwendung einer Zusammensetzung nach einem der Ansprüche 1 bis 45 zur Herstellung einer auf den Keratinsubstanzen haftenden Abscheidung und/oder für das schnelle Schminken von Keratinsubstanzen.

49. Verwendung einer Mascara nach Anspruch 46 zur schnellen Verdickung und/oder Verlängerung der Wimpern.

50. Verwendung der Kombination mindestens eines ersten Polymers mit einer gewichtsmittleren Molmasse unter 100.000, das ein Polymergerüst mit wiederkehrenden Kohlenwasserstoffeinheiten besitzt, die mindestens eine nicht als Seitenkette vorliegende Amideinheit und mindestens eine als Seitenkette vorliegende Fettkette und/oder mindestens eine endständige Fettkette aufweist, welche gegebenenfalls funktionalisiert sind, 6 bis 120 Kohlenstoffatome aufweisen und an die Amideinheiten gebunden sind, und mindestens eines Wachses in einer physiologisch akzeptablen, als Wachs-in-Wasser-Emulsion vorliegenden Zusammensetzung zur Herstellung eines haftenden Depots auf den Keratinsubstanzen und/oder zum schnellen Schminken von Keratinsubstanzen und/oder zur schnellen Verdickung und/oder Verlängerung der Wimpern.

51. Verwendung nach Anspruch 50, **dadurch gekennzeichnet, dass** das erste Polymer ein Polyamid ist, das endständige Gruppierungen mit Estergruppe aufweist, die eine Kohlenwasserstoffkette mit 10 bis 42 Kohlenstoffatomen besitzen.

52. Verwendung nach einem der Ansprüche 50 oder 51, **dadurch gekennzeichnet, dass** das erste Polymer eine gewichtsmittlere Molmasse von 1.000 bis 30.000 aufweist.
